# EUROPEAN PATENT APPLICATION

(11) **EP 4 600 962 A1**
(43) Date of publication of application: **13.08.2025**
(21) Application number: 24156911.0
(22) Date of filing: 09.02.2024
(51) Int. Cl.: G16B 15/10, G16B 40/20

(54) **METHOD AND SYSTEM FOR ESTIMATING A BINDING AFFINITY OF A TRIPLEX FORMING OLIGONUCLEOTIDE**

(71) Applicant: Universität Regensburg, 93053 Regensburg (DE)
(72) Inventor: KUGLER, Fabian, 93047 Regensburg (DE); BABL, Sabrina, 93053 Regensburg (DE); LÄNGST, Gernot, 81479 München (DE)
(74) Representative: Hofmann, Matthias

(57) **Abstract**

The present invention provides a method for estimating a binding affinity of a triplex forming oligonucleotide, TFO, at a triplex target site, TTS, of a double-stranded DNA, dsDNA, the method comprising:
- obtaining a plurality of TFO features based on sequence information of the TFO and a plurality of TTS features based on sequence information of the TTS, and
- processing the plurality of TFO features and the plurality of TTS features in a pre-trained model to estimate the binding affinity.

## Description

### TECHNICAL FIELD

The present invention relates to a method for estimating a binding affinity of a triplex forming oligonucleotide, a method for training a machine learning model and a data processing system, in particular a system distributed over a computer network, configured to carry out such a method.

The present invention also relates to a computer-readable storage medium storing program code, the program code comprising instructions for carrying out such a method.

### BACKGROUND

Nucleic acid triplexes have emerged as a pivotal area of study in molecular biology, particularly in the exploration of regulatory mechanisms for gene expression. The concept of triplex forming oligonucleotides (TFOs) harnesses the ability of single-stranded DNA or RNA molecules to specifically bind to double-stranded DNA (dsDNA), forming triple-helical structures. These structures occur naturally within the genome but can also be synthetically designed to target genomic sequences of interest.

TFOs recognize and bind to major groove regions of dsDNA at sites referred to as triplex target sites (TTSs). This binding can interfere with transcription, replication, and other nucleic acid processes within cells, making TFOs potentially powerful tools for gene modulation. Biological applications of TFOs stretch from basic research, where they are used to probe DNA structure and function, to clinical settings, where they hold promise for personalized medicine and gene therapy by selectively targeting and regulating disease-related genes.

A fundamental aspect of developing TFO-based technologies is the characterization of the binding affinity between a TFO and its corresponding TTS-the stronger and more specific the binding, the more effective and efficient the TFO is at modulating gene function. However, measuring binding affinity through traditional experimental means can be time-consuming and resource-intensive. Moreover, the high specificity required for successful binding to the dsDNA target site demands a precise understanding of the TFO sequence and the TTS.

Thus, there is a need for a method that allows prediction of the binding affinity between a TFO and its corresponding TTS, yet avoids experimental efforts.

### SUMMARY OF THE INVENTION

A first aspect of the present invention provides a method for estimating a binding affinity of a triplex forming oligonucleotide, TFO, at a triplex target site, TTS, of a double-stranded DNA, dsDNA, the method comprising:
- obtaining a plurality of TFO features based on sequence information of the TFO and a plurality of TTS features based on sequence information of the TTS, and
- processing the plurality of TFO features and the plurality of TTS features in a pre-trained model to estimate the binding affinity.

The method of the first aspect has the advantage that the binding affinity of different TFO and TTS pairs can be estimated without requiring physical measurements.

This step of obtaining a plurality of TFO features preferably involves analyzing the sequence of the TFO to identify characteristics or patterns that might influence its ability to bind to the TTS. Features may comprise the nucleotide sequence of the TFO, length, the potential for forming secondary structures, GC content, and/or any modifications that have been made to the oligonucleotide.

The step of obtaining a plurality of TTS features preferably involves analyzing the sequence of the TTS in the dsDNA. Features may comprise the nucleotide sequence of the TTS, length, potential secondary DNA structures, GC content, and/or any peculiarities of the DNA which might affect binding.

In the step of processing features in a pre-trained model, the pre-trained model refers to a computational model which has already been trained on a dataset where the binding affinities between TFOs and TTSs are known. By training on this data, the model learns to identify patterns and correlations between the features and binding affinities. Different kinds of machine learning techniques can be used here, including neural networks or support vector machines, among others.

The pre-trained model thus uses this information to estimate (predict) the binding affinity-or the strength of the interaction-between a given TFO and TTS based on the features identified in the preceding steps. The output is a quantitative measure of how tightly the TFO is likely to bind to the TTS, which can be critical for applications such as gene regulation, mapping of chromosomal structure, or gene therapy.

In a first implementation of the method according to the first aspect, the plurality of TFO features and/or the plurality of TTS features comprise(s):
- a frequency of adenine, thymine, guanine, and/or cytosine,
- a frequency of dinucleotides guanin-cytosine, guanine-adenine, cytosine-thymine and/or guanine-thymine,
- a frequency of k-mer motifs, and/or
- a length of the TFO and/or a length of the TTS,
wherein in particular the frequencies are relative frequencies.

Frequency of adenine (A), thymine (T), guanine (G), and/or cytosine (C) refers to how often each nucleotide occurs within the TFO sequence and the TTS sequence, respectively. For example, if the TFO has 10 adenines in a 20-base long sequence, the (relative) frequency of adenine would be 50%.

Frequency of dinucleotides guanine-cytosine (G-C), guanine-adenine (G-A), cytosine-thymine (C-T), and/or guanine-thymine (G-T): This feature does not look at individual nucleotides but at pairs of nucleotides, for example, how often a G is followed by a C or an A by a T. The frequency of other k-mer motifs is considered in the same manner. These are indications of the base-pairing rules that typically govern DNA structure and stability. In the context of the TFO and TTS, these pairs would not necessarily be base pairing within the strand; rather, it is a measure of dinucleotide frequency within the sequence.

The length of the TFO and/or the length of the TTS simply refers to the number of nucleotides present in the sequence of the TFO and the TTS, respectively.

It is also mentioned that the frequencies may be 'relative frequencies'. A relative frequency is the ratio of the observed frequency of a feature to the total number of possible observations. In the context of DNA sequences, this may mean the observed frequency of a nucleotide or nucleotide pair divided by the total length of the TFO or TTS sequence.

Experiments have shown that these features have been proven particularly easy to calculate, yet giving good results in the estimated binding affinities.

In a further implementation of the method according to the first aspect, the TFO features and/or the TTS features comprise one or more structural features, wherein preferably the one or more structural features comprise one or more of HelT, rise, roll, shift, slide, tilt, buckle, opening, ProT, shear, stagger, stretch, MGW and EP.

The terms used herein are preferably defined as follows:
- Helical Twist (HelT): The angle between adjacent base pairs in the DNA helix.
- Rise: The vertical distance between consecutive base pairs along the helical axis.
- Roll: The tilting of the base pairs relative to each other, which contributes to the curvature of the DNA.
- Shift: The lateral displacement between consecutive base pairs, typically perpendicular to the helical axis.
- Slide: The lateral displacement between consecutive base pairs, typically parallel to the helical axis.
- Tilt: The rotation of base pairs around the axis that runs through the center of each base pair (in contrast to helical twist).
- Buckle: The bending of the base pair itself, causing deviation from planarity.
- Opening: The degree to which base pairs are not facing each other fully (they seem "open").
- Propeller Twist (ProT): The rotation of one base with respect to the other base in a base pair, causing a propeller-like shape.
- Shear: The relative displacement of bases in opposite strands of DNA along the axis perpendicular to the base pair planes.
- Stagger: Describes axial displacement between base pairs from opposite strands (not inline with each other).
- Stretch: Changes in the distance between phosphates across the helix, affecting the width of the DNA.
- Minor Groove Width (MGW): The width of the minor groove in the DNA helix, an important factor in protein-DNA interactions.
- Electric Properties (EP): Potentially refers to electrostatic properties of the DNA which may comprise charge distribution, potential, and/or other electric properties influencing the DNA structure.

Including these features in the input for the pre-trained model means that the model does not only consider the linear sequence of nucleotides but also how the physical and structural configuration of the DNA and TFO can influence their binding affinity. Such considerations may provide a more comprehensive and accurate model for predicting the binding of TFOs to their target DNA sequences, particularly in cases where secondary and tertiary structural features play a significant role in the binding interaction.

These structural features provide important quantitative information about the structure that may be used in the machine-learning model.

In a further implementation of the method according to the first aspect, the method comprises a further step of calculating one or more structural features based on the sequence information of the TFO and/or the TTS.

The structural features can be determined efficiently and with high accuracy based on a computation based on the sequence information. For this purpose, existing software libraries can be used.

In a further implementation of the method according to the first aspect, the method further comprises a step of creating, before obtaining the plurality of TFO features and/or the plurality of TTS features based on the sequence information, a padding of the sequence information such that padded sequence information of a uniform length is obtained. This has the advantage that predictions can be made even in cases where the training data used for training the machine learning model have a different length compared to the TFO/TTS sequences, for which estimates should be made.

Since biological sequences like DNA or RNA can vary in length, but many machine learning models require fixed-size inputs, padding can here be used to standardize the sequences without losing significant information.

In an embodiment, the padding may be performed as follows:
1. Sequence Length Determination: First, a uniform length is determined. This length may be based on the longest sequence in the dataset or a predetermined length that fits the model requirements.
2. Padding Process: For sequences shorter than this uniform length, additional nucleotides (or a placeholder character) are added to the sequence to reach the required (uniform) length. Padding can be performed at the beginning (pre-padding), at the end (post-padding) of sequences, or both, depending on the convention or requirement of the specific analysis or machine learning model being used.
3. Uniform Dataset Creation: Once all sequences are padded to the same (uniform) length, a dataset with uniform-sized sequences is obtained, allowing for a more consistent feature extraction process and input into the machine learning model.

The padding ensures that all TFOs and TTSs are represented in a consistent way, making it easier for the computational models to process and analyze the data without bias toward longer or shorter sequences. Different kinds of padding (such as nucleotide identity or placeholder characters) can be used, and the padding can be applied in different locations (pre, post, or both).

In a further implementation of the method according to the first aspect, the pre-trained model has been trained using biophysical measurements of the binding affinity. The biophysical measurements can thus serve as ground-truth data.

In a further implementation of the method according to the first aspect, the pre-trained model comprises a Support Vector Machine, SVM, regression model. Experiments have shown that in the present context SVM regression yields particularly high accuracy estimates compared to other machine learning methods. Additionally or alternatively, the pre-trained model may comprise a neural network, random forests, and/or gradient boosting machines.

In a further implementation of the method according to the first aspect, training of the pre-trained model has been performed on oligonucleotides with different lengths, content of guanine and cytosine, and base sequences, in particular for three different motifs Purin, Pyrimidin and a mixture of Purin and Pyrimidin.

The following variations of the training data may be used:
1. Oligonucleotides with different lengths: The training dataset includes oligonucleotides (short DNA or RNA molecules) of variable lengths. This helps the model learn to predict binding affinities for TFOs regardless of their size, which is important since the length of an oligonucleotide can influence its binding properties.
2. Content of guanine and cytosine (GC Content): GC content refers to the percentage of bases in a DNA or RNA molecule that are either guanine (G) or cytosine (C). GC content can affect the stability of the oligonucleotide-DNA complex because G-C base pairs have three hydrogen bonds, compared to just two in adenine-thymine (A-T) base pairs. This means that a higher GC content typically results in a higher melting temperature and greater stability. Training the model with TFOs and TTSs of various GC contents allows the model accounting for these stability differences when predicting binding affinities.
3. Base sequences: The machine learning model is also trained on sequences consisting of different nucleotide arrangements. Since the sequence composition can drastically change the shape and reactive properties of a TFO and its target site, diversified training sequences are essential for the model to learn how different sequences affect binding affinity.
4. Three different motifs: The machine learning model is specifically trained on data for three distinct motifs:
   - Purine motifs: Sequences rich in purines (adenine (A) and/or guanine (G)) are known to form certain types of triplex structures with high affinity.
   - Pyrimidine motifs: Sequences rich in pyrimidines (cytosine (C) and/or thymine (T)).
   - Mixed motifs: Sequences that contain a mix of purine and pyrimidine nucleotides. This variety enables the pre-trained model to predict how different motifs might influence the binding affinity of a TFO to a TTS.

Training the model on such diverse datasets ensures that it captures a wide range of potentially influential features and interaction nuances, leading to more accurate and generalizable predictions of binding affinity across different TFO and TTS sequences. This enriched training background allows the model handling various real-world scenarios effectively when being used to estimate binding affinities of new TFOs and TTSs.

In a further implementation of the method according to the first aspect, the method further comprises:
- identifying possible TTSs in a specific gene, in particular a gene that is dysregulated leading to a disease, and
- for each possible TTS, estimating binding affinities for a plurality of TFOs and determining a TFO with the highest binding affinity as a most suitable TFO.

1. Identifying possible TTSs (Triplex Target Sites) in a specific gene: In this step, the method involves scanning the sequence of a gene to pinpoint potential TTS-regions where a TFO could bind to form a stable triplex DNA structure. Given that the gene in question is dysregulated leading to a disease, this implies a therapeutic interest, wherein the modulation of gene expression through TFO binding could provide clinical benefits.
2. Estimating binding affinities for a plurality of TFOs: For each TTS identified, the model is then used to predict how strongly various TFOs could bind to these sites. This step may involve the systematic application of the previously outlined method to generate binding affinity estimates using sequence-based features of both the TFOs and the TTSs.
3. Determining a TFO with the highest binding affinity: After generating binding affinity predictions for each pairing of TFOs with the identified TTSs, the method comprises comparing these estimates to find out which particular TFO has the strongest predicted interaction with the TTS. The TFO with the highest estimated binding affinity is then selected as the most suitable TFO for that site.

This process is preferably executed in an iterative manner and may involve screening numerous TFO candidates to find the optimal one for each TTS within the gene. The ultimate goal is to select a TFO that will most effectively bind to the TTS within the disease-causing gene, potentially altering its activity in a desired manner (for example, inhibiting the expression of a gene contributing to disease pathology).

This implementation can be used for applications such as gene therapy, where TFOs are used to target genes for regulation at the transcriptional level, either to reduce or to enhance their expression depending on the therapeutic needs. Identifying TFOs with high binding affinity is crucial, as stronger binding may result in a more effective and durable regulation of gene expression.

In a further implementation of the method according to the first aspect, the method further comprises:
- identifying possible TTSs in a specific gene, in particular a dysregulated gene leading to a disease, and
- for each possible TTS, generating all possible sequences according to Hoogsteen base pairing and TFO motif, and
- using sequence alignment to search for the generated possible sequences in specific RNA (e.g. IncRNA and/or viral RNA).

In a further implementation of the method according to the first aspect, the method further comprises using a TFO for which a high binding affinity has been estimated for forming a triplex for targeted overexpression or knockdown of a gene. The above has shown particularly useful.

A second aspect of the present invention provides a method for training a machine learning model for estimating a binding affinity of a triplex forming oligonucleotide, TFO, at a triplex target site, TTS, of a double-stranded DNA, dsDNA, in particular a machine learning model for use in a method of the first aspect or one of its implementations, the method comprising:
- obtaining a plurality of TFO features based on sequence information of a plurality of TFOs and obtaining a plurality of TTS features based on sequence information of a plurality of TTSs,
- obtaining corresponding binding affinities of pairs of the plurality of TFOs and the plurality of TTSs, and
- training the machine learning model using the plurality of TFO features, the plurality of TTS features and the corresponding binding affinities.

The method of this second aspect refers to the process of creating (training) a machine learning model that may be used to estimate the binding affinity of a triplex forming oligonucleotide (TFO) to a triplex target site (TTS) on double-stranded DNA (dsDNA). The machine learning model developed through this process can then be applied in the method of estimating binding affinities according to the first aspect and its implementations.
1. Obtaining a plurality of TFO features: The first step involves collecting sequence-related features from a group of TFOs. These features may comprise aspects such as nucleotide composition, frequency of k-mer motifs, length, GC content, sequence specific DNA structures (HelT, rise, roll, shift, slide, tilt, buckle, opening, ProT, shear, stagger, stretch, MGW and EP), triplex motifs, potential secondary structures that could influence a TFO's ability to bind to a TTS.
2. Obtaining a plurality of TTS features: Similarly, this step involves collecting sequence-related features from a group of TTS sequences present in dsDNA. Again, the focus is on aspects that could affect the binding affinity of TFOs to these target sites.
3. Obtaining corresponding binding affinities: This step requires gathering empirical data on the binding affinities between each TFO and TTS pair. Binding affinity can be measured through various experimental techniques like surface plasmon resonance, electrophoretic mobility shift assays, or other biophysical or biochemical methods. These affinities provide the target output that the machine learning model is trained to estimate.
4. Training the machine learning model: With the features and corresponding binding affinities obtained, the final step is to train the machine learning model. During training, the model learns to associate specific combinations of TFO and TTS features with their experimentallymeasured binding affinities. Various machine learning algorithms could be used, including, but not limited to, support vector machines, neural networks, random forests, or gradient boosting machines.

The overall objective of the training process is to enable the model to accurately predict the binding affinity of any new TFO-TTS pair based on their sequence information. Once trained, this model can be used to estimate the binding affinity for novel TFOs or TTSs, facilitating the design and testing of TFOs for research or therapeutic applications.

A third aspect of the present invention refers to a data processing system, in particular a system distributed over a computer network, configured to carry out a method of one of the first aspect of the present invention, the second aspect of the present invention and one of their implementations.

A fourth aspect of the present invention refers to a computer program comprising instructions which, when the computer program is executed by a computer, cause the computer to carry out the steps of the method of one of the first aspect of the present invention, the second aspect of the present invention and one of their implementations.

A fifth aspect of the present invention refers to a computer-readable storage medium storing computer program code, the computer program code comprising instructions that when executed by a processor carry out the method of the second aspect or one of the implementations of the second aspect.

### BRIEF DESCRIPTION OF THE DRAWINGS

To illustrate the technical features of embodiments of the present invention more clearly, the accompanying drawings provided for describing the embodiments are introduced briefly in the following. The accompanying drawings in the following description are merely some embodiments of the present invention, modifications on these embodiments are possible without departing from the scope of the present invention as defined in the claims.
- FIG. 1: shows an overview of Watson-Crick base pairing, triplex motifs and Hoogsteen base pairing.
- FIG. 2: shows DNA shape features, which are relevant also for TFO-TTS binding.
- FIG. 3: illustrates a prediction of the binding affinity of triplex forming oligonucleotides (TFOs) and dsDNA (TTSs), including an overview of the most important parameters for the prediction.
- FIG. 4: shows a workflow of the screen for the best possible TFO sequences for gene regulation by means of triplex structures.

### DETAILED DESCRIPTION OF EMBODIMENTS

The foregoing descriptions are only implementation manners of the present invention, the scope of the present invention is not limited to this. Any variations or replacements can be easily made through person skilled in the art. Therefore, the protection scope of the present invention should be subject to the protection scope of the attached claims.

The morphological and physiological properties (phenotype) of cells are based on the part of the genetic information which is read out from the DNA at this time. As a result of the process of gene expression, an RNA copy, the so-called messenger RNA (mRNA), which is subsequently translated into proteins, emerges from the DNA information in eukaryotic cells. Diseases can arise as a result of defective DNA (mutations) or as a result of excessive or reduced production (gene expression) of proteins. If mutations occur in genes, this can lead to defective proteins (missense mutations) or to the absence of proteins (nonsense mutation). In the case of altered gene expression, the protein is functional, but the concentration is increased or reduced, which has led to the phenotype caused by the disease.

In order to investigate a change in gene expression caused by the disease, mRNA is sequenced and the number of mRNA molecules formed per gene is determined. If the mRNA concentration of "healthy" and "diseased" cells is compared, differentially expressed genes can be identified.

Important constituents of a gene are the coding region (information for mRNA or protein) and the promoter. This is located upstream of the coding sequence and is required for binding the necessary regulatory proteins of gene expression. Furthermore, there are so-called enhancer regions which can likewise enhance or suppress gene expression by recruiting transcription factors. Together with the enhancer, the promoter is one of the regulatory elements of the DNA, the accessibility and interaction of which with further proteins causes gene expression to be identified.

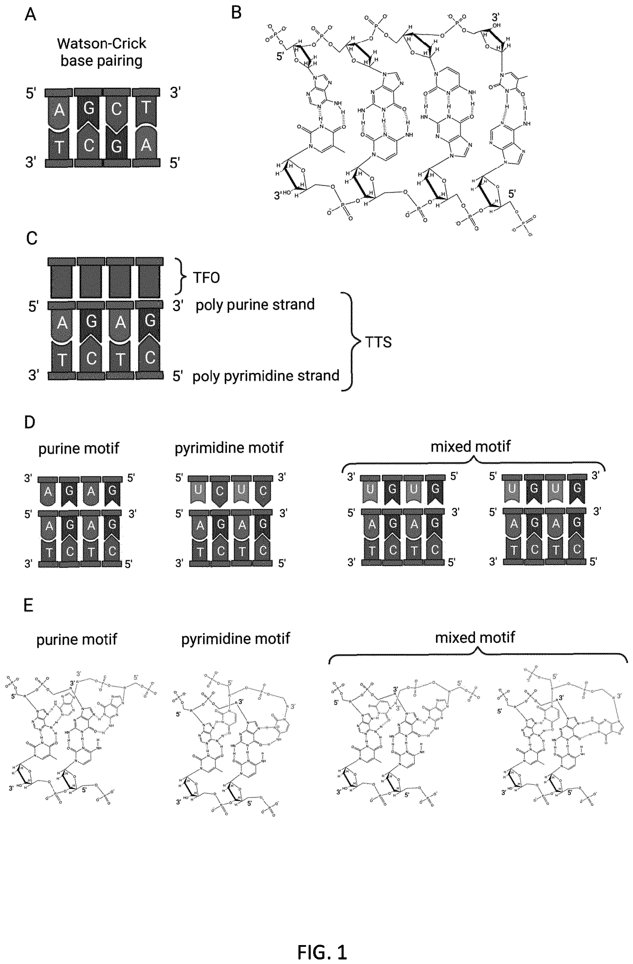

The DNA is present as a double helix (Watson-Crick base pairing, FIGs. 1 A and B). Under certain conditions, a third RNA/DNA strand can specifically attach to the DNA double strand (dsDNA) and form a three-stranded nucleic acid (triplex). These structures occur in cells and have gene regulatory functions. In this case, hydrogen bonds are formed between the oligonucleotides (short DNA or RNA molecules) and the polypurine strand (DNA sequence with the bases adenine and guanine) of the dsDNA according to the Hoogsteen base pairing (FIGs. 1 C to E). These oligonucleotides are called "triplex forming oligonucleotides" (TFOs) and attach spatially to the large groove of the DNA double helix. The possible binding sites in the DNA are called "triplex target sites" (TTSs). In order to be able to form hydrogen bonds, the sequence of the TFO and of the TTS must match according to the Hoogsteen base pairing. The combination of TTS and TFO is called "triple helix" or triplex. If triplexes occur in regulatory regions of genes, this can lead to activation or repression of gene expression. Triplexes in coding regions can block the transcription complex (proteins which transcribe DNA into RNA) and thus lead to reduced gene expression.

The formation of triplexes is currently theoretically predicted only according to the Hoogsteen base pairing. Biophysical measurements of the binding affinity show that these predictions are not reliable and triplex regions do not form despite perfect Hoogsteen base pairing. However, the precise prediction of homologous TFO sequences and the associated binding sites in the dsDNA (TTSs) is a necessary prerequisite for the design of artificial triplex elements which could be used as therapeutics in vivo.

One aim of the present invention comprises finding regulatory TTSs in medically relevant genes and the subsequent identification of potential regulatory RNA for molecular biological research purposes and artificial applications. In certain embodiments, gene regulatory therapeutics and functional biosensors, inter alia, can be developed from these predicted RNA sequences.

FIG. 1 shows an overview of Watson-Crick base pairing, triplex motifs and Hoogsteen base pairing. A) shows the Watson-Crick base pairing (A-T and G-C). The two DNA single strands attach to one another in reverse complementary fashion. The orientation of the DNA strands results from the sugar backbone (deoxyribose). The numbering of the carbon atoms in the sugar ring indicates the direction (5' C atom on the phosphate group and 3' C atom on the hydroxyl group). B) Chemical structure of the schematic DNA. Hydrogen bonds (red) hold the single strands together and form on the complementary bases. C) Scheme of a triplex structure in which the TFO (here RNA) attaches to the polypurine strand of the TTS. D) Possible triplex motifs for the same TTS sequence. Depending on the composition (purine bases, pyrimidine bases or "mixed") of the TFO sequence, triplexes with different orientation form. The orientation is antiparallel for purine TFOs (cf. TFO with polypurine TTS) or parallel for pyrimidine TFOs. TFOs with "mixed" motif can bind both antiparallel and parallel. E) Chemical structures of the schematic TFOs. Only the first two bases are shown. The TFO (here RNA, in blue) attaches to the TTS (black). New hydrogen bonds to the hydrogen bonds of the complementary DNA single strands (red) also form hydrogen bonds between the polypurine strand of the TTS and the TFO (green).

The present disclosure provides a pipeline ("Triplex Affinity Prediction", TAP) to precisely predict triplexes. For this purpose, the inventors use a combination of biophysical experiments and machine learning. The Hoogsteen base pairing alone is not sufficient to predict triplex structures. Despite optimal base pairing, some TFOs in binding affinity measurements by means of microscale thermophoresis (MST) show insufficient binding affinity to actually occur in cells. In order to find essential properties for triplex formation, affinities of oligonucleotides with different lengths, GC content and base sequence were measured for the three possible motifs (purine, pyrimidine and "mixed").

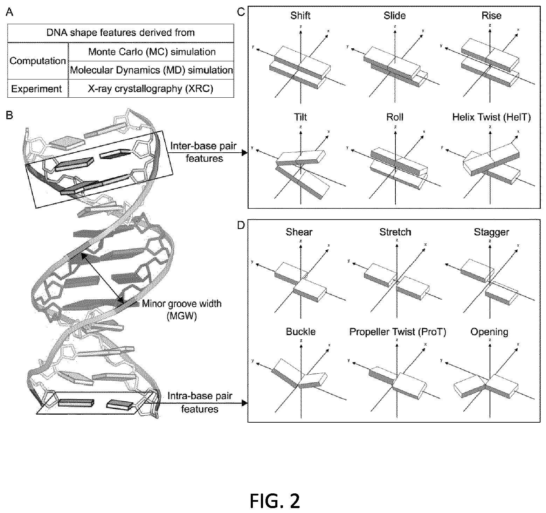

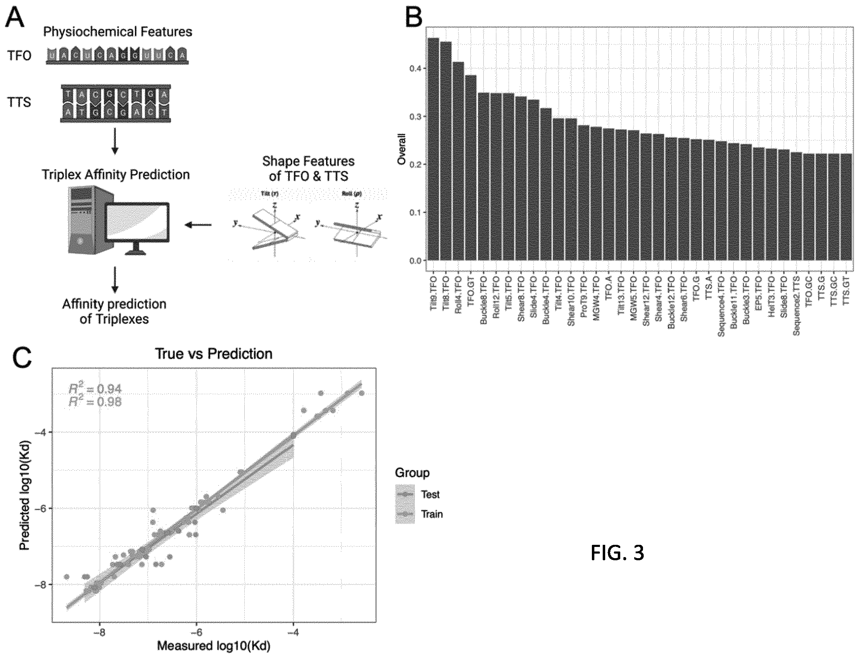

Biophysical measurements were combined with the analysis of the three-dimensional structure of TTSs and TFOs (FIG. 2) in a machine learning model to predict the binding affinity in silico. Depending on the sequence, the DNA forms a predictable 3D structure which can be calculated. In combination with the sequence of the oligonucleotides and the physicochemical properties, the binding affinity can be predicted with high precession (accuracy of 94% in the test data) (FIGs. 3 A and C). The form and structure of the DNA are identified by the sequence of the individual bases; these interact with one another via steric or electronic interactions (FIG. 3 B). An example of this is the different number of hydrogen bonds between G-C and AT (3 or 2).

FIG. 2 shows DNA form factors: A) methods to determine the DNA form factors. B) Schematic representation of a DNA double helix. C) Examples of inter-base pair factors. These determine the spatial alignment of two consecutive complementary base pairs. D) shows intra-base pair factors. Here, the spatial alignment between the complementary bases is determined.

Further important factors which determine the binding affinity are the GC content (proportion of guanine and cytosine) of the TFO and of the TTS and the length of the TFO and of the corresponding TTS (FIG. 3 B). With increasing GC content, the binding affinity for all TFO motifs decreases. For homologous triplex formation, the TFO requires a minimum length of 13 nucleotides, otherwise the length has no further influence. In cells, the binding of TFOs to dsDNA is used for regulatory purposes. The sequences of TFO and dsDNA must always match perfectly for this purpose. The deviations between TFO and TTS (according to Hoogsteen base pairing) are called mismatches and influence the binding affinity. The binding affinity is identified to a different extent depending on the position of the mismatch and the substituted bases. The influence strength correlates with the structural changes of the TTS and of the TFO which arise as a result of the mismatches. The binding affinity is likewise identified by the TFO motif, thus the same TTS can be bound differently well by all possible TFOs. Specifically in the case of "mixed" motifs, the orientation between TFO and TTS plays an important role, since this likewise identifies the binding affinity.

FIG. 3 shows a prediction of the binding affinity of triplex forming oligonucleotides (TFOs) and dsDNA (TTS). A) shows an overview of the "Triplex Affinity Prediction" model (TAP). This uses physiochemical properties such as length and GC content of the TFO and of the TTS sequence. Of both sequences, the shape is additionally determined on the basis of 14 parameters and this likewise also enters the model. The model was trained and tested with biophysical data. B) shows the most important parameters for the affinity prediction. The form and structure of the TTS and of the TFO have a greater influence on the binding affinity than the physiochemical properties. C) shows an accuracy in the prediction for the training data set (blue) of 98% and for the test data set (red) of 94%.

Double-stranded DNA can specifically attach a third nucleic acid strand in the large groove. This third strand is bound by means of the Hoogsteen base pairing rules on account of the structure. The orientation of the nucleic acid strands results from the sugar backbone (deoxyribose). The numbering of the carbon atoms in the sugar ring indicates the direction (5' C atom on the phosphate group and 3' C atom on the hydroxyl group). Depending on the composition (purine bases, pyrimidine bases or "mixed") of the TFO sequence, triplexes with different orientation will form. The orientation is antiparallel for purine TFOs (cf. TFO with polypurine TTS) or parallel for pyrimidine TFOs. TFOs with "mixed" motifs can bind both antiparallel and parallel (see FIG. 1).

The table used for training preferably contains columns which reflect the sequence-specific properties of the nucleic acids. For the triplex forming oligonucleotides (TFOs), the length of the sequence (TFO.Length), is the length of the nucleotides A, T, G, C (TFO.A, TFO.T, TFO.G, TFO.C) and the length of the dinucleotides GC, GA, CT, GT (TFO.GC, TFO.GA, TFO.CT, TFO.GT) and frequency of k-mer motifs in the TFO sequences, in each case expressed as probability. For the triplex target sites (TTSs), the length of the sequence (TTS.Length) is the length of the nucleotides A, T, G, C (TTS.A, TTS.T, TTS.G, TTS.C) and the length of the dinucleotides GC, GA, CT, GT (TTS.GC, TTS.GA, TTS.CT, TTS.GT) and frequency of k-mer motifs in the TTS sequences, in each case expressed as probability. The resulting table thus contains a summary of the above-described sequence-specific properties of the two DNA sequence sets.

An adjustment of the data length may be useful, in particular in cases where training data are only present up to a certain length of base pairs.

A "padding" function preferably obtains two parameters: "length" (the current length of the data frame) and "name" (the name of the sequence). The maximum length (n.max) is set as the TFOs and TTSs are investigated up to a maximum of 29, since only triplex pairs up to a length of 29 bases were analyzed biophysically and these data serve as training data set. A table (df.pad) is created which has the difference between the maximum length and the current length. Each cell in the data frame is initialized with NA (Not Available). The column names of this table are identical to the column names of the table with the sequence-specific properties. A column named "position" is created and filled with values from the current length +1 up to the maximum length. A column named "SequenceID" is created and filled with the name of the sequence, again as often as the difference between the maximum length and the current length.

In summary, this function thus creates a data frame with missing values and supplements it so that it reaches a certain maximum length. This is necessary if it is to be ensured that all data frames have the same length in one analysis.

The function preferably obtains two parameters: "sequence" (the DNA sequence) and "name" (the name of the sequence). The function then calls the "get.shape" function in order to then calculate DNA structure parameters for the specified sequence. These parameters comprise various aspects of the DNA structure, such as helicity HelT, rise, roll, shift, slide, tilt, buckle, opening, ProT, shear, stagger, stretch, MGW and EP. These parameters are preferably calculated for each position in the sequence. The calculated structure parameters are then converted into a table (data.frame) which contains various columns for each structure parameter, the position within the sequence, the sequence ID (name of the sequence) and the actual sequence. The actual sequence of the nucleic acids is converted into a number sequence by means of "onehot encoding". Here, a number is assigned to each of the four bases (A, T, C, G). For some structure parameters (HelT, roll, rise, shift, slide, tilt), an NA value is added to the vector at the end. This is necessary since these structure parameters are calculated between consecutive bases, as a result of which the vectors obtained are shorter by one value than the actual sequence. In the table, each column must have the same number of rows.

For the determination of the 3D structure, the R package DNAshapeR is preferably used to determine all available features ("HelT", "rise", "roll", "shift", "slide", "tilt", "buckle", "opening", "ProT", "shear", "Stagger", "Stretch", "MGW", "EP").

Based on the sequence names, the tables are reformatted, so that all parameters per TTS or TFO sequence are in one row. In this format, the TTS and TFO properties can then be combined in a large table by stringing the columns together. A table which contains, row by row, all parameters per triplex pair thus arises.

The columns with the information of the sequence names and the sequence position (index within the sequence) have no information about the sequences and are removed from the table in order not to affect machine learning. In order to obtain an exclusively numerical table (matrix), all previously introduced NAs are converted into "o". This is necessary in order to carry out subsequent calculations (normalization and scaling). The sequence columns (only numbers in (1,2,3,4)) and the percentages of nucleotides, dinucleotides and k-mer motifs are excluded from the normalization, since these columns are normalized and lie in the same value range for all samples. The remaining columns are centered and scaled based on the corresponding mean value and the standard deviation of the training data.

With the above-described table, the affinity for each triplex pair can now be determined.

A machine model for the prediction of the variable Kd (logarithmic Kd values) is generated based on the features in the normalized table of the triplex pair properties. 918 parameters are available per triplex pair. First, the common logarithm of the experimentally determined Kd values is calculated, which is to be predicted by the model. 85% of the experimentally tested data are used for the training and the remaining data as test data set. The data were randomly divided into training set or test set. For the training and the subsequent test, the column with the Kd values is excluded from the "input" table. As training control, a cross-validation with repetitions was used, wherein 10 folds and 5 repetitions are set. A support vector machine (SVM) model with linear kernel function was created and trained. For this, the R package caret* can preferably be used. The model is trained with the training data and checked using the cross-validation and the specified training control and with the test data set.

Some applications of the triplex prediction for generating gene regulatory RNA sequences are presented below.

In gene therapy, human diseases are treated specifically by introducing genetically altered DNA. For this, the genetic material has to be transported through the cell membrane and into the cell nucleus with the aid of so-called vectors. In the development of new therapies, general requirements have to be taken into account: Target cells have to be specifically recognized and bound (avoidance of off-target effects). Vector-based immune reactions have to be avoided. Specific uptake into the target cells is important. The stability of the vector and genetic material within the cell and target organism (e.g. enzymatic degradation and physiological conditions) has to be ensured. Transfer of the genetic material into the cell nucleus and specific gene expression within the cell is important.

A large part of the vectors used to date is based on viruses, as a result of which the natural ability of these viruses to penetrate cells can be advantageously used. Viruses also have the ability to survive for a long time within a cell and either to activate, inhibit or modify the natural defensive mechanisms of the cell. As a result, in most cases the enzymatic degradation of the viral vectors after uptake into the cell is prevented (Chen et al., 2018). The simplicity and small size of the viral genome make viral vectors a suitable tool in gene therapy, since they can be easily modified (Lukashev & Zamyatnin, 2016). However, in the case of these viral vectors there is the potential risk of integration into the genome of the target cells, which can lead to activation of oncogenes or inactivation of tumor suppressors and thus cause cancer. In addition, the immune system in the body reacts to viral vectors, as a result of which they are rapidly degraded before they reach the target cells (Cornetta et al., 1990). For this reason, they are more suitable for ex vivo therapies.

In order to avoid the disadvantages mentioned, non-viral vectors have also been intensively researched and tested over time. In contrast to viral vectors which can naturally penetrate the cell membrane, non-viral vectors have to overcome several obstacles in order finally to transport the DNA into the cell nucleus. Non-viral vectors do not have a natural ability to recognize target cells, and therefore ligands have to be added which specifically bind to surface proteins of the target cells (for example antibodies). In order to facilitate uptake into the cytoplasm, the negatively charged DNA is condensed with positively charged polymers or lipids. This enables cellular uptake by receptor-mediated endocytosis, but also leads to non-specific interactions with extracellular components or cells. This can be problematic since binding to plasma proteins activates the reticulohistiocytic system, a part of the immune system, and thus a large part of the injected material is degraded (Yin et al., 2014; Zhang et al., 2012). On account of such problems, non-viral gene therapy approaches generally have a lower efficiency. Since the non-viral vector is taken up by the target cell by endocytosis, the enzymatic degradation of the DNA in the lysosome has to be subsequently prevented. For this purpose, lipopolyplex vectors have been developed which are hybrids of positively charged lipids and positively charged polymers. These interact with the negatively charged DNA, condense it and thus protect it from nucleases (Yin et al., 2014). However, these vectors have a certain toxicity, which makes their application in vivo difficult without further optimization (Grandinetti et al., 2012). Since DNA itself is too large to be transported through the nuclear pore complex into the cell nucleus, an additional nuclear localization sequence (NLS) has to be added to the vector system in order to enable active transport and the start of gene expression (Zhang et al., 2012).

For the application of the triplex prediction, the "triplex finding tool" (TFT) has been developed, which first of all searches for all possible TTSs in specific genes by means of PATO (Naki Amatria-Barral et al., 2023). Starting from the found TTSs, the best possible (most homologous) TFO sequence is determined by means of implemented TAP.

Alternatively, all possible sequences (according to Hoogsteen base pairing and TFO motif) can be generated for the found TTSs. By means of sequence alignment, it is then possible to search for these TFO sequences in specific RNA (e.g. IncRNA, viral RNA). The correspondences can then be identified by means of parameters, for example according to mismatches and length. Based on this filter, the TTS and TFO pairs are then generated and their binding affinity is predicted by means of implemented TAP.

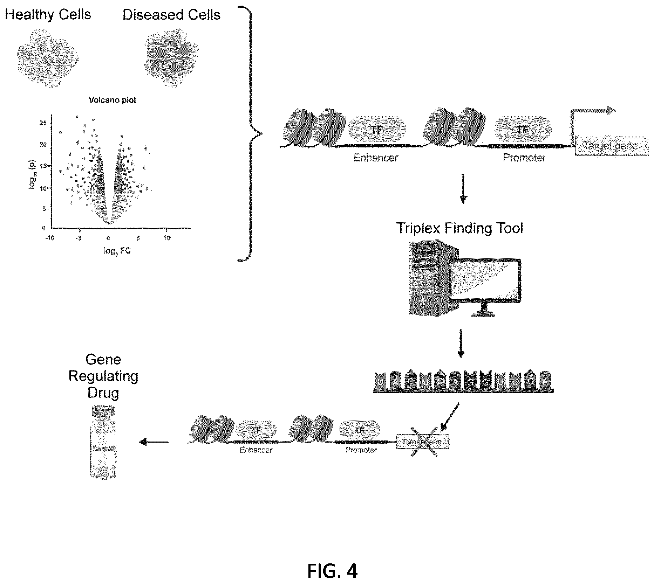

FIG. 4 shows a workflow of the screen for the best possible TFO sequences for gene regulation by means of triplex structures. First, differentially expressed disease-causing genes are determined. Subsequently, for each gene, TTS motifs are searched for in the regulatory and coding regions. The "triplex finding tool" then generates TFO sequences with high affinity for the found TTS sequences. The TFO sequence then forms a triplex with the TTS and can block or increase the gene expression depending on the region and modification of the TFO sequence. The TFO can then be used as an RNA therapeutics.

In order to investigate the function of certain genes or their proteins, genes of interest are often investigated in cell culture by means of knockdown or knockout experiments. In this case, the amount of the protein to be investigated is reduced or completely removed. In addition, the amount of the protein can be increased (overexpression).

For the overexpression, the corresponding gene is inserted into a plasmid which is then transfected into the cells. This increases the gene copy number, which leads to a higher expression. In the case of a knockdown, a small interfering RNA (siRNA) can be inserted into a plasmid which is then transfected into the cells. The inserted RNA is complementary to the mRNA of the corresponding gene. As a result, an RNA-RNA hybrid then forms and the mRNA cannot be translated into protein. Generating plasmids through cloning, propagation in bacteria (such as Escherichia coli), and subsequent purification can be a time-consuming process for these two methods. For the application of triplex-based gene regulation for overexpression or knockdown, the specific gene only has to be analyzed by means of TFT and TFO sequences are obtained which then form triplexes and cause the desired gene regulation. The TFO can then be transfected without plasmid.

Possible target genes for a therapy can be determined by means of sequencing of mRNA and the subsequent analysis of differentially expressed genes between "healthy" and "diseased" cells. The regulatory (promoter and enhancer) and the coding regions of these genes can then be investigated by means of the TFT and possible TTSs can be found. Based on the polypurine strand of the TTS, highly homologous TFO sequences are created and the binding affinity is predicted. The best possible TFO sequence is selected mainly via the binding affinity but also via the number of "non-specific" binding sites in the entire human genome. Here, triplex structures in the coding region are more likely to reduce gene expression, while triplexes in the promoter regions and corresponding binding sites for enhancer increase gene expression. In this way, any "errors" in gene expression can be regulated and the phenotype caused by the disease can be reduced. Currently available gene therapy is mainly aimed at exchanging defective genes and proteins for functional ones. For this, so-called transgenes are introduced into somatic cells by means of viral vectors. As a result, the cell can use the transgene as a template and express error-free protein. This approach functions only in the case of mutations in the genes, but not in the case of an altered expression of non-mutated genes.

With the aid of epigenetic gene therapy, the expression of genes can be altered, genes being activated or inactivated by means of modifications of histones (e.g. methylation/demethylation). These modifications are, however, per se not gene-specific; for this, these mechanisms have to be coupled to proteins which recognize and bind specific DNA sequences (for example zinc finger proteins (ZNFs)). A general, non-specific mechanism is thus coupled to a sequence-specific protein.

A further possibility in gene therapy for lowering the expression is the use of siRNA which binds to the corresponding mRNA and leads to a degradation of the latter, without a protein being synthesized.

An advantage of triplex-mediated gene regulation is the direct approach to the DNA before translation into mRNA. As a result, the gene regulation is already regulated from the start, as a result of which the efficiency is increased. A general advantage of triplexes is that the TFOs can both recognize the specific DNA sequence and affect the gene regulation. In contrast to conventional epigenetic gene therapy, a highly specific mechanism is used in this case, since the TFOs bind only specifically and the regulation of the gene expression is affected by the binding.

Compared to available gene therapies, gene regulation by means of triplex structures does not require any vectors, since the TFOs are very small and can penetrate into cells and cell nuclei without auxiliaries. As a result, vector-based immune reactions and the risk of incorporation of viral elements of the vectors into the genome are avoided, therefore unwanted oncogene activation and deactivation of tumor suppressor genes are pretended. The specificity is given by the sequence of the TFO and can be determined in silico (analysis of the entire genome for possible binding sites). This enables in vivo gene therapy without risks of permanent alteration of the human genome.

One prerequisite of triplex-mediated gene therapy is the prediction of possible TFO candidates for specific genes/disease patterns and the analysis of possible homologous target sites in the genome. As a result, the number of sequences to be tested is reduced and it is possible to ensure that only specific bindings between TFO and the genes to be treated occur.

Triplex formation allows the specific detection of DNA, antibodies and proteins, but physiological conditions such as pH or heavy metal ions can also be detected. In this case, physical signals (e.g. voltage change, fluorescence) are identified and measured by the structural change of the triplex formation. These sensor methods are distinguished by high sensitivity, specificity and a high signal-to-noise ratio (Hu et al., 2017).

In general, the triplex formation can also be measured via a change in electrical voltage. In this case, a TFO is immobilized on an electrode. The TFO can then specifically bind DNA from a sample, as a result of which the voltage changes and this can be measured in real time (Petralia et al., 2020). This approach is particularly interesting as a biosensor if real-time data are required.

This type of diagnostics can already be used to detect SARS-CoV-2 RNA. The conventional reverse transcription quantitative polymerase chain reaction (RT-qPCR) for detecting SARS-CoV-2 genome is time-consuming and requires costly reagents. In the more recent triplex-based detection method, the viral RNA is complementarily bound with a "labeled" oligonucleotide and subsequently a triplex forms with the hairpin structure of further oligonucleotide functioning as anchor. The detection is then carried out, for example, via fluorescence-elec-tromobility shift assays (EMSA) or, if fluorescence is used, via color reactions (cf. antibody rapid test) (Avino et al., 2022). With the described methods, specific pairs of oligonucleotides and TTS can be generated and the binding affinity can be predicted, which can then be used to detect particular DNA or RNA.

## Claims

1. A method for estimating a binding affinity of a triplex forming oligonucleotide, TFO, at a triplex target site, TTS, of a double-stranded DNA, dsDNA, the method comprising:
- obtaining a plurality of TFO features based on sequence information of the TFO and a plurality of TTS features based on sequence information of the TTS, and
- processing the plurality of TFO features and the plurality of TTS features in a pre-trained model to estimate the binding affinity.

2. The method of claim 1, wherein the plurality of TFO features and/or the plurality of TTS features comprise(s):
- a frequency of adenine, thymine, guanine, and/or cytosine,
- a frequency of dinucleotides guanin-cytosine, guanine-adenine, cytosine-thymine and/or guanine-thymine,
- a frequency of k-mer motifs, and/or
- a length of the TFO and/or a length of the TTS,
wherein in particular the frequencies are relative frequencies.

3. The method of one of the previous claims, wherein the TFO features and/or the TTS features comprise one or more structural features, wherein preferably the one or more structural features comprise one or more of HelT, rise, roll, shift, slide, tilt, buckle, opening, ProT, shear, stagger, stretch, MGW and EP.

4. The method of claim 3, wherein the method comprises a further step of calculating one or more structural features based on the sequence information of the TFO and/or the TTS.

5. The method of one of the previous claims, further comprising a step of creating, before obtaining the plurality of TFO features and/or the plurality of TTS features based on the sequence information, a padding of the sequence information such that padded sequence information of a uniform length is obtained.

6. The method of one of the previous claims, wherein the pre-trained model has been trained using biophysical measurements of the binding affinity.

7. The method of one of the previous claims, wherein the pre-trained model comprises a Support Vector Machine, SVM, regression model, a neural network, random forests, and/or gradient boosting machines.

8. The method of one of the previous claims, wherein training of the pre-trained model has been performed on oligonucleotides with different lengths, content of guanine and cytosine, and base sequences, in particular for three different motifs Purin, Pyrimidin and a mixture of Purin and Pyrimidin.

9. The method of one of the previous claims, further comprising:
- identifying possible TTSs in a specific gene, in particular a dysregulated gene leading to a disease, and
- for each possible TTS, estimating binding affinities for a plurality of TFOs and determining a TFO with the highest binding affinity as a most suitable TFO.

10. The method of one of claims 1 to 8, further comprising:
- identifying possible TTSs in a specific gene, in particular a dysregulated gene leading to a disease, and
- for each possible TTS, generating all possible sequences according to Hoogsteen base pairing and TFO motif, and
- using sequence alignment to search for the generated possible sequences in specific RNA (e.g. lncRNA and/or viral RNA).

11. The method of one of claims 9 and 10, further comprising using a TFO for which a high binding affinity has been estimated for forming a triplex for targeted overexpression or knockdown of a gene.

12. A method for training a machine learning model for estimating a binding affinity of a triplex forming oligonucleotide, TFO, at a triplex target site, TTS, of a double-stranded DNA, dsDNA, in particular a machine learning model for use in a method of one of the previous claims, the method comprising:
- obtaining a plurality of TFO features based on sequence information of a plurality of TFOs and obtaining a plurality of TTS features based on sequence information of a plurality of TTSs,
- obtaining corresponding binding affinities of pairs of the plurality of TFOs and the plurality of TTSs, and
- training the machine learning model using the plurality of TFO features, the plurality of TTS features and the corresponding binding affinities.

13. A data processing system, in particular a system distributed over a computer network, configured to carry out a method of one of the previous claims.

14. A computer program comprising instructions which, when the computer program is executed by a computer, cause the computer to carry out the steps of the method of one of claims 1 to 12.

15. A computer-readable storage medium storing computer program code, the computer program code comprising instructions that when executed by a processor carry out the method of one of claims 1 to 12.
